# EUROPEAN PATENT SPECIFICATION

(11) **EP 3 287 468 B1**
(45) Date of publication and mention of the grant of the patent: **16.09.2020**
(21) Application number: 15889516.9
(22) Date of filing: 23.04.2015
(51) Int. Cl.: C07K 7/08, C07K 1/00, C12N 15/00, A61K 38/00, A61P 9/00, A61P 9/12

(54) **TWO CONOTOXIN PEPTIDES, PREPARATION METHODS THEREFOR, AND APPLICATIONS THEREOF**
ZWEI CONOTOXINPEPTIDE, HERSTELLUNGSVERFAHREN DAFÜR UND ANWENDUNGEN DAVON
DEUX PEPTIDES DE CONOTOXINE, PROCÉDÉS DE PRÉPARATION ASSOCIÉS ET APPLICATIONS ASSOCIÉES

(43) Date of publication of application: 28.02.2018
(73) Proprietor: BGI Shenzhen, Shenzhen, Guangdong 518083 (CN)
(72) Inventor: LIU, Jie, Shenzhen Guangdong 518083 (CN); LIN, Zhilong, Shenzhen Guangdong 518083 (CN); WEN, Bo, Shenzhen Guangdong 518083 (CN); TONG, Ting, Shenzhen Guangdong 518083 (CN); MO, Fen, Shenzhen Guangdong 518083 (CN)
(74) Representative: Ricker, Mathias
(86) International application number: PCT/CN2015/077301
(87) International publication number: WO 2016/169027

(56) References cited:
- CN-A- 1 297 454
- CN-A- 101 557 820
- DAI XIAN-DONG ET AL: "Isolation and Characterization of Conotoxin bt5a from Conus betulinus", CHINESE JOURNAL OF NATURAL MEDICINES, vol. 8, no. 2, 20 March 2010 (2010-03-20), pages 132-136, XP002785093, ISSN: 1672-3651

SEQUENCE LISTING

<110> 旮詁貌湮價秖旃噶埏

<120> 謗笱鄆蹟凴匼踽｝・桊掘源楊摯茼蚚

<130> PCT150013PPC

<160> 4

<170> PatentIn version 3.3

<210> 1
<211> 11
<212> PRT
<213> 漆鰍莉肭俱鄆蹟ㄗConus betulinus ㄘ

<400> 1

Ser Glu Cys Cys Ile Arg Asn Phe Leu Cys Cys
1               5                   10

<210> 2
<211> 19
<212> PRT
<213> 漆鰍莉肭俱鄆蹟ㄗConus betulinus ㄘ

<400> 2

Ala Leu Gln Arg Tyr Trp Ala Lys Ser Leu Cys Cys Pro Glu Asp Arg
1               5                   10                  15

Trp Cys Cys

<210> 3
<211> 33
<212> DNA
<213> 漆鰍莉肭俱鄆蹟ㄗConus betulinus ㄘ

<400> 3
tctgaatgct gcattagaaa tttcctctgc tgt                                    33

<210> 4
<211> 57
<212> DNA
<213> 漆鰍莉肭俱鄆蹟ㄗConus betulinus ㄘ

<400> 4
gccctacaaa gatattgggc gaaatccttg tgctgcccag aagataggtg gtgctgt 57

## Description

### Technical field

The present invention relates to the field of biomedical technology and, in particular, to two novel conotoxin peptides ω-CPTx-btl1 and ω-CPTx-btl2, polynucleotides encoding the two peptides, constructs, expression vectors and host cells comprising the polynucleotides, natural extraction and synthetic methods of the peptides, and the pharmaceutical uses of the peptides.

### Background art

Calcium ion channel is a kind of transmembrane protein widely distributed in excitatory cells, which can regulate entry of extracellular Ca²⁺ into the cytoplasm and participate in a series of Ca²⁺-dependent physiological activities such as hormonal and neurotransmitter releases, muscle contraction and cell signaling. According to different sensitivities to voltage, calcium ion channels can be divided into low-voltage activated calcium ion channel (LVA) or T-type calcium channel (Cav3.x) and high-voltage activated calcium ion channel, wherein HVA calcium channel includes L-type (Cav1.x), P/Q-type (Cav2.1), N-type (Cav2.2) and R-Type (Cav2.3) calcium channels. The Cav1.x channel participates in muscle contraction and cell secretion and the Cav2.x participates in neurotransmitter release and neuronal excitatory regulation. The calcium channel consists of α-subunit having the main function and β, γ and δ subunits having the auxiliary function.

Calcium channel blockers are widely found in the venom of animals, such as centipedes, spiders and the like. Many drugs of calcium channel blockers such as dihydropyridine small molecules have been developed. Typical drugs include the selective calcium ion channel blocker nifedipine, which is mainly used in the clinical treatment of hypertension, coronary heart disease, arrhythmia, angina pectoris, cerebrovascular diseases and the like, and belongs to a class of targeted drugs having the most wide and highly frequent application currently.

However, relatively few drugs of polypeptide calcium channel blockers have been currently developed, and as an example, Ziconotide is a class of N-type calcium channel blockers and is useful for the treatment of refractory chronic pain. Polypeptide calcium channel blockers, due to their many advantages, such as high selectivity, high lipophilicity and high safety, will become a hotspot in the future development of drugs of calcium channel blockers.

Hypertension is one of the most common cardiovascular diseases in the world today and is a major risk factor for cardiovascular and cerebrovascular diseases. The research results published by Center for Chronic Disease Prevention and Control of China CDC show that the number of hypertensive patients in China has exceeded 330 million, and on average, one in every 3 adults is a hypertensive patient. There are five types of antihypertensive drugs at first-line, namely, calcium channel blockers (CCB), angiotensin converting enzyme inhibitors (ACE I), angiotensin receptor blockers (ARB), diuretics and β-receptor blockers. Among them, calcium channel blockers act as the main force of the retail market of antihypertensive drugs currently, and reduce blood pressure mainly through blocking the calcium channels on myocardium and vascular smooth muscle cell membranes, inhibiting influx of extracellular calcium ions so as to decrease the intracellular calcium ion levels, and thereby lead to the decreased contraction of the cardiovascular and other tissue smooth muscles and vasodilatation, which thereby reduces blood pressure.

Conus is a class of toxic animals widely distributed in the tropical marine, and the venom thereof contains a large number of polypeptide ion channel active substances, such as ω-Conotoxin, which can specifically block the voltage-sensitive calcium ion channel, and thus it is an important natural resource for development of antihypertensive drugs of polypeptide calcium channel blockers.

CN 101 557 820 A describes ω-conotoxin peptides from Conus flavidus that bind to N-type calcium channels and have, e.g., the amino acid sequence CKGTGKSCSRIAYNCCTGSCRSGKC. CN 1 297 454 A describes an ω-conotoxin peptide that selectively blocks calcium channels in which the fourth loop between cysteine residues 5 and 6 comprises the amino acid sequence SGTVGR. Dai Xian-Dong et al. (Chinese Journal of Natural Medicine 8(2):132-136, 2010) discloses a peptide called conotoxin Bt5a isolated from Conus betulinus and having the amino acid sequence SXCCIRNFLCC.

### Summary

It is an object of the present invention to provide two novel conotoxin peptides ω-CPTx-btl1 and ω-CPTx-btl2, polynucleotides encoding the two peptides, constructs, expression vectors and transformed host cells comprising the polynucleotides, natural extraction and synthetic methods of the peptides, and the pharmaceutical uses of the peptides.

The present invention achieves the above object by the following technical scheme:
In a first aspect, the present invention provides a conotoxin peptide ω-CPTx-btl1, which is a polypeptide having the amino acid sequence shown in SEQ ID NO: 1; and the amino acid sequence of the polypeptide contains two pairs of disulfide bonds, which is required for this conotoxin peptide to play its inhibitory activity on calcium ion channels.

In a second aspect, the present invention provides a conotoxin peptide ω-CPTx-btl2, which is a polypeptide having the amino acid sequence shown in SEQ ID NO: 2; and the amino acid sequence of the polypeptide contains two pairs of disulfide bonds, which is required for the conotoxin peptide to play its inhibitory activity on calcium ion channels.

The inventors of the present invention firstly extracted and identified the conotoxin peptides ω-CPTx-btl1 and ω-CPTx-btl2 of the present invention from the venom ducts of *Conus betulinus* native to Hainan, and the amino acid sequences thereof are SECCIRNFLCC (as shown in SEQ ID NO: 1) and ALQRYWAKSLCCPEDRWCC (as shown in SEQ ID NO: 2) respectively, and the molecular weights thereof are 1286.5 daltons and 2327.7 daltons respectively.

In a third aspect, the present invention provides a polynucleotide encoding the conotoxin peptide as described in the first aspect or the second aspect.

In a specific embodiment, the polynucleotide encoding the conotoxin peptide ω-CPTx-btl1 having the amino acid sequence as shown in SEQ ID NO: 1 has a nucleotide sequence as shown in SEQ ID NO: 3, i.e., the nucleotide sequence of TCTGAATGCTGCATTAGAAATTTCCTCTGCTGT; the polynucleotide encoding the conotoxin peptide ω-CPTx-btl2 having the amino acid sequence as shown in SEQ ID NO: 2 has a nucleotide sequence as shown in SEQ ID NO: 4, i.e., the nucleotide sequence of

GCCCTACAAAGATATTGGGCGAAATCCTTGTGCTGCCCAGAAGATAGGTGG
TGCTGT.

In a fourth aspect, the present invention provides a nucleic acid construct comprising the polynucleotide as described in the third aspect, and one or more control sequences operably linked thereto and being able to direct the production of the polypeptide in an expression host.

In a fifth aspect, the present invention provides an expression vector comprising the nucleic acid construct as described in the fourth aspect.

In a sixth aspect, the present invention provides a transformed cell into which the nucleic acid construct as described in the fourth aspect or the expression vector as described in the fifth aspect is transformed.

In a seventh aspect, the present invention provides a conotoxin peptide as described in the first aspect or the second aspect for use in the treatment of a cardiovascular disease, in particular a cardiovascular disease selected from hypertension, angina pectoris, coronary heart disease, or arrhythmia.

The inventors have studied the biological activity of the two conotoxin peptides ω-CPTx-btl1 and ω-CPTx-btl2 of the present invention. Specifically, the effects of the two conotoxin peptides ω-CPTx-btl1 and ω-CPTx-btl2 of the present invention on the dorsal root ganglion cells (DRG cells) ion channels were detected by using a whole-cell patch clamp method. In the experiment, two depolarization stimuli are given: the first is at from -90 mV to -30 mV for 250 ms, the second is at from -60 mV to 0 mV for 250 ms, the interval between the two stimuli is 500 ms, and the LVA calcium channel and HVA calcium channel are recorded simultaneously. By comparison analysis with the activity of Verapamil (which is a calcium channel blocker, and is useful for the treatment of hypertension, angina pectoris, arrhythmia, cerebrovascular diseases, angiospasm of the finger, abdominal pain, esophageal achalasia, migraine, pulmonary hypertension and the prevention of premature delivery) as a positive control, its effect of inhibiting calcium ion channels and its subsequent application were determined.

The inventors of the present invention have found that the two conotoxin peptides ω-CPTx-btl1 and ω-CPTx-btl2 of the present invention both have the effect of suppressing the current of the high-voltage activated calcium ion channels (HVA) and their inhibitory effect is comparable to that of Verapamil as a positive control.

Since the two conotoxin peptides ω-CPTx-btl1 and ω-CPTx-btl2 of the present invention both have the effect of suppressing the current of the high-voltage activated calcium ion channels (HVA), they can be used for the treatment of diseases such as hypertension, angina pectoris and coronary heart disease.

In an eighth aspect, the present invention provides a pharmaceutical composition comprising the conotoxin peptide as described in the first aspect or the second aspect and a pharmaceutically acceptable carrier.

In a ninth aspect, the present invention provides a method for producing the conotoxin peptide as described in the first aspect or the second aspect, comprising:
(1) synthesizing the linear peptide of the conotoxin peptide according to the amino acid sequence as shown in SEQ ID NO: 1 or 2 by solid-phase chemical synthesis, preferably by fluorenylmethoxycarbonyl solid-phase chemical synthesis;
(2) performing oxidative refolding of the linear peptide obtained in step (1) with glutathione method.

In addition, the two conotoxin peptides ω-CPTx-btl1 and ω-CPTx-btl2 of the present invention can also be obtained by extraction from a natural organism. Specifically, the inventors of the present invention extracted toxin polypeptides from the venom ducts of *Conus betulinus* native to Hainan and then performed the steps of separation and identification to obtain the polypeptides; preferably, strong cation exchange high performance liquid chromatography is used for the separation; preferably, mass spectrometry is used for the identification of polypeptides.

In a specific embodiment, the conotoxin peptide having the amino acid sequence as shown in SEQ ID NO: 1 or 2 was extracted and identified by the following steps:
Collecting *Conus betulinus* native to Hainan, taking venom ducts and extracting toxin polypeptide therefrom were firstly performed. After reductive alkylation treatment with dithiothreitol (DTT) and iodoacetamide (IAM), the toxin polypeptides were fractionated by using strong cation exchange high performance liquid chromatography (SCX-HPLC) and then detected by using nano-high performance liquid chromatography-mass spectrometry (NanoLC-MS/MS) for mass spectrum of the peptide. The resulting mass spectrometry data was parsed and analyzed bioinformatically to obtain the complete amino acid sequence of the conotoxin polypeptide.

### Beneficial effect

The two conotoxin peptides ω-CPTx-btl1 and ω-CPTx-btl2 of the invention are available from natural active animal resource, belong to calcium channel blockers, and have the advantages of high selectivity, high lipophilicity and high safety compared with traditional small molecule medicine. As they have simple structures, are easy to be artificially synthesized, and can effectively inhibit the current of calcium channel and the inhibitory activity is higher than that of Verapamil, they can be widely used in the treatment of calcium ion channel related diseases; for example, in clinical practice, they have great potential in the treatment of hypertension, heart disease, angina pectoris and other diseases.

### Description of the drawings

Figure 1 shows the mass spectrometric identification result of the sequence of the conotoxin peptide ω-CPTx-btl1 of the present invention;
Figure 2 shows the mass spectrometric identification result of the sequence of the conotoxin peptide ω-CPTx-btl2 of the present invention;
Figure 3 shows the inhibitory effect of 10 µM conotoxin peptide ω-CPTx-btl1 on high-voltage activated calcium channels;
Figure 4 shows the inhibitory effect of 10 µM conotoxin peptide ω-CPTx-btl2 on high-voltage activated calcium channels;
Figure 5 shows the inhibitory effect of 10 µM Verapamil on high-voltage activated calcium channels.

### Detailed description

For the purpose of understanding the present invention, the present invention is exemplified as follows. It will be apparent to those skilled in the art that the examples are merely illustrative of the invention and should not be construed as a specific limitation of the present invention.

### Example 1 Extraction and identification of the two conotoxin peptides ω-CPTx-btl1 and ω-CPTx-btl2 of the present invention

### 1. Extraction and reductive alkylation of Conus venom

Four *Conus betulinus* native to Hainan were dissected after smashing their shells, and then the venom ducts thereof were clipped and the Conus venoms were collected. Bradford method was performed to determine the protein concentration in the venom, which was 6.48 mg/ml. The total amount of protein was 0.5 mg. DTT at a final concentration of 1 mM was added and reacted at 56 °C for 1 h. After reduction and cooling to room temperature, IAM at a final concentration of 55 mM was added to react in dark room at room temperature for 45 min.

### 2. Enrichment of toxin polypeptides

The conotoxin polypeptides obtained as above were then subjected to Strata-X C18 column to enrich the polypeptides in Conus venoms. The enrichment by Strata-X C18 was performed according to the following standard procedure: 1) adding 1ml of methanol to activate the column; 2) adding 1 ml 0.1% FA to balance the column; 3) loading 1 ml venom sample, washing with buffer (5% ACN + 0.1% FA), repeating the wash for 3 times; 4) eluting with 100% ACN, collecting the eluent. The molecular weights of the enriched polypeptides were detected by MALDI-TOF-MS.

### 3. Sequence identification of the conotoxin polypeptides

240 µg peptide mixture was fractionated by a SCX-HPLC (Shimadzu) system: buffer A: 10 mM KH₂PO₄ in 25% ACN, pH 3.5; buffer B: further containing 500 mM potassium chloride on the basis of buffer A; Flow rate: 1 ml/min; elution procedure: 0-40% linear binary gradient of buffer B for 10 minutes, 40-90% of buffer B for 2 minutes, 90% of buffer B for 3 minutes; absorbance detection was performed at 214 nm; as a result, a total of 10 fractions were collected through gradient elution. The collected fractions were demineralized by C18 solid phase extraction column (Strata-X, Phenomenex) and then reconstituted with 30 µl of 0.1% formic acid for nanoLC-MS / MS analysis.

### 4. NanoLC-MS / MS analysis

LC/MS was from Shimadzu's nano HPLC chromatograph system and AB Sciex's Triple TOF 5600 mass spectrometer system. Each pre-separated polypeptide component was separated by a self-made Ultimate capillary analysis column which had a length of 12 cm, an inner diameter of 75 µm and was filled with Welch Materials brand XB-C18 column material with a particle size of 3 µm and a pore diameter of 120 µm at a flow rate of 300 nl / min. The injection volume for detection was 25 µl and the concentration of Buffer B was evenly increased from 5% to 45% for 40 min for a gradient elution. For mass spectrum acquisition, the electrospray voltage was 2.5kV, the auxiliary air pressure was 30PSI, the sheath gas pressure was 15PSI, and the source temperature was 150 °C. The first-order mass spectrum was acquired using a high-resolution mode greater than or equal to 30000. The valence state of parent ions in the range of 2 charges to 5 charges was selected for acquisition of the second-order mass spectrum. After one scanning of the first-order mass spectrum, 30 second-order mass spectrometric fragmentations can be conducted continuously, so that 30 scannings of the second-order spectrum daughter ions can be completed in 250 ms, and more than 120 sheets of the second-order spectrums can be generated per second. The total cycle time was 3.3 seconds.

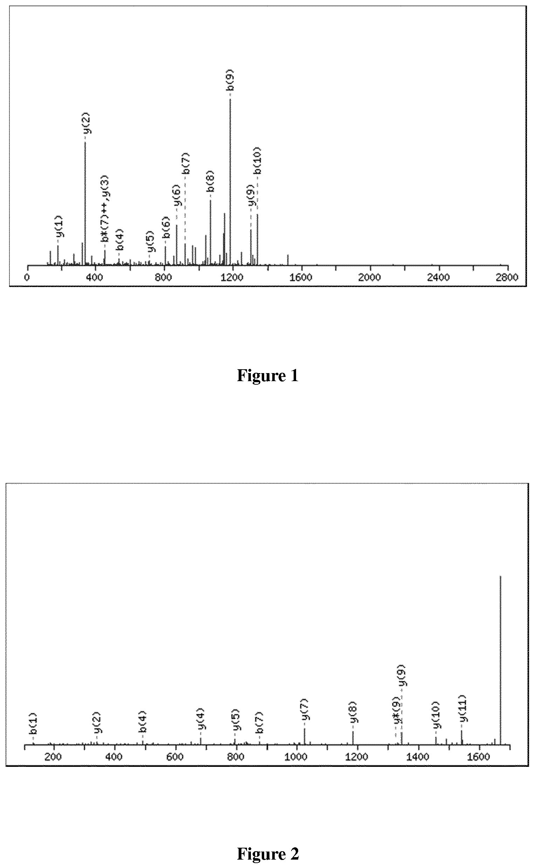

In the obtained mass spectrometry raw data, the sequence search and alignment results of the mass spectrum corresponding to the conotoxin peptides ω-CPTx-btl1 and ω-CPTx-btl2 of the present invention were shown in Fig. 1 and Fig. 2, respectively.

### 5. Data analysis

The mass spectrometry raw data obtained by nanoLC-MS/MS detection was format converted into MGF, which then was subjected to data search and identification by Mascot search software. In the obtained polypeptide sequences, the ω-CPTx-btl1 polypeptide having a full length amino acid sequence of SECCIRNFLCC (as shown in SEQ ID NO: 1) and ω-CPTx-btl2 polypeptide having a sequence of ALQRYWAKSLCCPEDRWCC (as shown in SEQ ID NO: 2) were selected by sequence feature analysis.

### Example 2 Chemical synthesis of the conotoxin peptides ω-CPTx-btl1 and ω-CPTx-btl2 of the present invention

The conotoxin linear peptide as shown in SEQ ID NO: 1 or 2 was synthesized by fluorenylmethoxycarbonyl (Fmoc) solid phase chemical synthesis (customized by GL Biochem (Shanghai) Ltd.), respectively.

The chemically synthesized polypeptide was refolded by using glutathione oxidative refolding, i.e.:
The polypeptide was dissolved in a solution at pH 7.4 containing 0.1 M Tris-HCl, 0.1 M NaCl, 5 mM GSH and 0.5 mM GSSG at a mass to volume ratio of 1:10 and reacted at 25 °C for 24 to 48 h. The refolding effect was detected by MALDI-TOF-MS.

### Example 3 Inhibitory activity of the conotoxin peptides ω-CPTx-btl1 and ω-CPTx-btl2 of the present invention on calcium ion channels

The inhibitory activity of the conotoxin peptides ω-CPTx-btl1 and ω-CPTx-btl2 of the present invention on calcium ion channels was detected by patch-clamp technique, respcetively. Specifically, the conotoxin peptides ω-CPTx-btl1 and ω-CPTx-btl2 as prepared and refolded in Example 2 were weighed, respectively, and detected for their effects on rat dorsal root ganglion (DRG) cell ion channels by whole-cell patch-clamp method. Verapamil was used as a positive control.

Intracellular fluid and extracellular fluid in patch-clamp method were configured as follows:
Extracellular fluid: 140 mM NaCl, 4 mM KCl, 1 mM MgCl₂, 2 mM CaCl₂, 5 mM D-Glucose monohydrate, 10 mM HEPES (pH = 7.4); intracellular fluid: 20 mM KCl, 110 mM potassium aspartate (KAspartic), 1 mM MgCl₂, 5 mM EGTA, 10 mM HEPES (pH = 7.2).

DRG cells (dorsal root ganglion cells immediately separated from SD rats and cultured) in the thermostatic incubator were taken out, and the culture medium in the culture dish was replaced with a well-balanced extracellular fluid at room temperature to prevent drastic changes in the temperature of the solution. The extracellular fluid was gently added with a pipette along the wall of the dish to prevent the cells shedding from the bottom of the dish. The cells in the extracellular fluid were placed under an inverted microscope for observation, and those cells with smooth cell membrane and homogeneous cytoplasm were selected, and subjected to the patch clamp test at a room temperature of 20-25 °C.

100 µl borosilicate glass blank was used as glass microelectrode material. A two-step drawing was performed by a drawing instrument to make the diameter of the electrode tip be about 1.5-3.0 µm, and the initial resistance of the glass microelectrode upon immersion into a liquid be 2-4 MΩ. The electrode was filled and installed followed by being moved below the liquid surface, and then a continuous positive pressure was immediately applied to ensure that the electrode tip was clean, that is, conducting liquid junction potential compensation. Under the inverted microscope, a microelectrode was moved over the selected cell and close to the cell, the positive pressure was removed and a slight negative pressure was applied for attraction. After forming a high impedance Giga-Ohm (GΩ) seal between the electrode and the cell membrane, an electrode fast capacitance compensation was conducted immediately.

The cell was then clamped at -60 mV, and a short and strong negative pressure was applied, thereby breaking the cell membrane clamped in the microelectrode rapidly and then performing cell low capacitance compensation. After forming the whole cell recording pattern, the cell was clamped at -90 mV for 4-6 min, and then the cell was subjected to two depolarization stimuli: the first one was at from -90 mV to -30 mV, for 250 ms, and the second one was at from -60 mV to 0 mV, for 250 ms, the interval between the two stimuli was 500 ms, and the LVA calcium channel and the HVA calcium channel were recorded at the same time. A polypeptide sample was added to the extracellular fluid to give an effective concentration of 10 µM. The changes in the recorded calcium channel current were observed simultaneously (the experiment was repeated three times and the result was an average of three replicates). The series resistance (Rs) was always constant within the range of <10 MΩ during the experiment, and the system series resistance compensation (Rseries compensation) was between 30 and 70%.

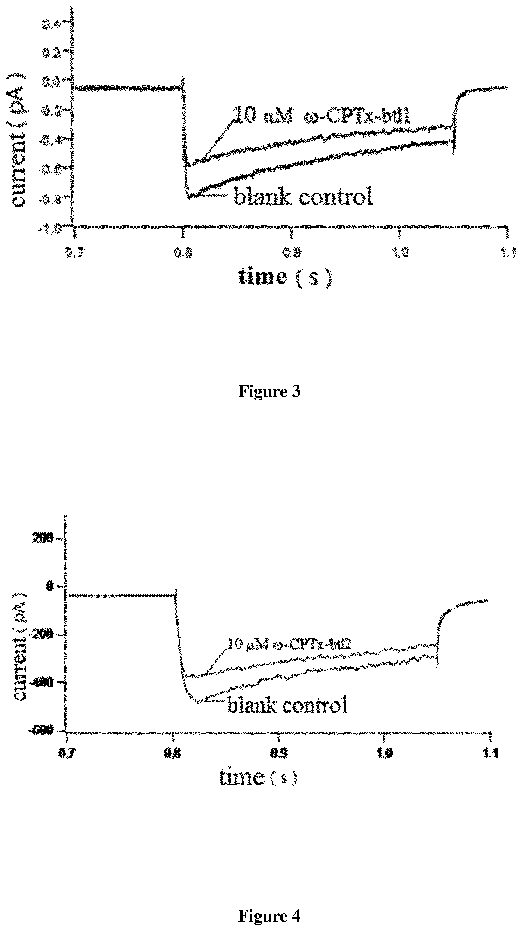

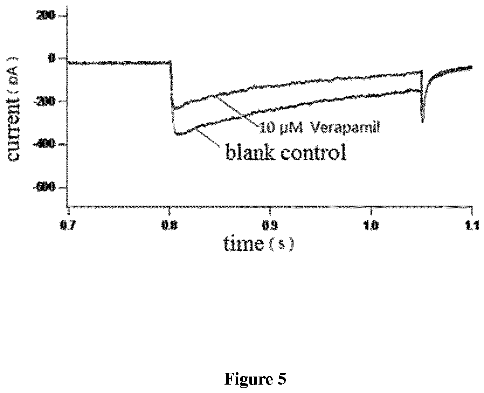

The detection results of the conotoxin peptides ω-CPTx-btl1 and ω-CPTx-btl2 were shown in Fig. 3 and Fig. 4, respectively, and the detection result of Verapamil was shown in Fig. 5. The inhibition rates of 10 µM of ω-CPTx-btl1 or ω-CPTx-btl2 on HVA calcium channel currents in DRG neuronal cells were 27.04% and 20.97%, respectively (as shown in Table 1). The inhibition rate of Verapamil on HVA calcium channel currents was 30.90%, higher than those of the peptides. The results showed that both conotoxin peptides ω-CPTx-btl1 and ω-CPTx-btl2 of the present invention can be used as candidates for antihypertensive drugs.

**Table 1. Patch clamp detection results of the inhibitory rates of the conotoxin peptides ω-CPTx-btl1 and ω-CPTx-btl2 on calcium ion channel**

| tested drugs | current before loading (pA) | current after loading (pA) | inhibition rate (%) |
|---|---|---|---|
| ω-CPTx-btl1 | -810.00 | -591.00 | 27.04 |
| ω-CPTx-btl2 | -487.48 | -385.26 | 20.97 |
| Verapamil | -343.00 | -237.00 | 30.90 |

## Claims

1. A conotoxin peptide ω-CPTx-btl1, which is a polypeptide having the amino acid sequence shown in SEQ ID NO: 1.

2. A conotoxin peptide ω-CPTx-btl2, which is a polypeptide having the amino acid sequence shown in SEQ ID NO: 2.

3. A polynucleotide encoding the conotoxin peptide according to claim 1 or 2.

4. A nucleic acid construct comprising the polynucleotide according to claim 3, and one or more control sequences operably linked thereto and being able to direct the production of the polypeptide in an expression host.

5. An expression vector comprising the nucleic acid construct according to claim 4.

6. A transformed cell into which the nucleic acid construct according to claim 4 or the expression vector according to claim 5 is transformed.

7. The conotoxin peptide according to claim 1 or 2 for use in the treatment of a cardiovascular disease.

8. The conotoxin peptide for use according to claim 7, wherein the cardiovascular disease is hypertension, angina pectoris, coronary heart disease, or arrhythmia.

9. A pharmaceutical composition comprising the conotoxin peptide according to claim 1 or 2 and a pharmaceutically acceptable carrier.

10. A method for producing the conotoxin peptide according to claim 1 or 2, comprising:
(1) synthesizing the linear peptide of the conotoxin peptide according to the amino acid sequence as shown in SEQ ID NO: 1 or 2 by solid-phase chemical synthesis, preferably by fluorenylmethoxycarbonyl solid-phase chemical synthesis;
(2) performing oxidative refolding of the linear peptide obtained in step (1) with glutathione method.

## Patentansprüche

1. Conotoxinpeptid ω-CPTx-btl1, welches ein Polypeptid ist, das die in SEQ ID NO: 1 gezeigte Aminosäuresequenz aufweist.

2. Conotoxinpeptid ω-CPTx-btl2, welches ein Polypeptid ist, das die in SEQ ID NO: 2 gezeigten Aminosäuresequenz aufweist.

3. Polynukleotid, welches das Conotoxinpeptid nach Anspruch 1 oder 2 codiert.

4. Nukleinsäurekonstrukt, welches das Polynukleotid nach Anspruch 3 und eine oder mehrere Kontrollsequenzen aufweist, die in einer wirkenden Weise damit verbunden sind und die in der Lage sind, die Erzeugung des Polypeptids in einem Expressionswirt zu steuern.

5. Expressionsvektor, welches das Nukleinsäurekonstrukt nach Anspruch 4 aufweist.

6. Transformierte Zelle, in welche das Nukleinsäurekonstrukt nach Anspruch 4 oder der Expressionsvektor nach Anspruch 5 transformiert worden ist.

7. Conotoxinpeptid nach Anspruch 1 oder 2 zur Verwendung bei der Behandlung einer Herz-Kreislauf-Erkrankung.

8. Conotoxinpeptid zur Verwendung nach Anspruch 7, wobei die Herz-Kreislauf-Erkrankung eine Hypertonie, eine Angina pectoris, eine koronare Herzkrankheit oder eine Arrhythmie ist.

9. Pharmazeutische Zusammensetzung, welche das Conotoxinpeptid nach Anspruch 1 oder 2 und einen pharmazeutisch verträglichen Träger aufweist.

10. Verfahren zur Herstellung des Conotoxinpeptids nach Anspruch 1 oder 2, welches umfasst:
(1) ein Synthetisieren des linearen Peptids des Conotoxinpeptids nach der Aminosäuresequenz, wie sie in SEQ ID NO: 1 oder2 gezeigt ist, durch eine chemische Festphasensynthese, und bevorzugt durch eine chemische Festphasensynthese mit Fluorenylmethoxycarbonyl;
(2) ein Durchführen einer oxidativen Rückfaltung des in Schritt (1) erhaltenen linearen Peptids mit dem Glutathion-Verfahren.

## Revendications

1. Peptide de conotoxine ω-CPTx-btl1, qui est un polypeptide présentant la séquence d'acides aminés représentée par SEQ ID NO : 1.

2. Peptide de conotoxine ω-CPTx-btl2, qui est un polypeptide présentant la séquence d'acides aminés représentée par SEQ ID NO : 2.

3. Polynucléotide codant pour le peptide de conotoxine selon la revendication 1 ou 2.

4. Construction d'acide nucléique comprenant le polynucléotide selon la revendication 3, et une ou plusieurs séquences de régulation liées de manière fonctionnelle à celui-ci et étant capables de diriger la production du polypeptide dans un hôte d'expression.

5. Vecteur d'expression comprenant la construction d'acide nucléique selon la revendication 4.

6. Cellule transformée dans laquelle la construction d'acide nucléique selon la revendication 4 ou le vecteur d'expression selon la revendication 5 est transformé.

7. Peptide de conotoxine selon la revendication 1 ou 2 pour une utilisation dans le traitement d'une maladie cardiovasculaire.

8. Peptide de conotoxine pour une utilisation selon la revendication 7, dans lequel la maladie cardiovasculaire est l'hypertension, une angine de poitrine, une maladie coronarienne ou une arythmie.

9. Composition pharmaceutique comprenant le peptide de conotoxine selon la revendication 1 ou 2 et un support pharmaceutiquement acceptable.

10. Procédé de production du peptide de conotoxine selon la revendication 1 ou 2 comprenant :
(1) la synthèse du peptide linéaire du peptide de conotoxine selon la séquence d'acides aminés telle que représentée par SEQ ID NO : 1 ou 2 par synthèse chimique en phase solide, de préférence par synthèse chimique en phase solide avec fluorényl-méthoxycarbonyle ;
(2) la mise en œuvre d'un repliement oxydatif du peptide linéaire obtenu dans l'étape (1) avec le procédé au glutathion.
